(19) **Europäisches Patentamt European Patent Office Office européen des brevets**

(11) **EP 3 395 408 B1**

(12) **EUROPEAN PATENT SPECIFICATION**

| | |
|---|---|
| (45) Date of publication and mention of the grant of the patent:<br>**04.03.2020 Bulletin 2020/10** | (51) Int Cl.:<br>**A61N 5/10** *(2006.01)* **G06K 9/00** *(2006.01)* |

(21) Application number: **18166313.9**

(22) Date of filing: **09.04.2018**

(54) **PATIENT MONITORING**

PATIENTENÜBERWACHUNG

SURVEILLANCE DE PATIENT

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR** | • **NILSING, Mattias**<br>**126 35 Hägersten (SE)**<br>• **KRONANDER, Åsa**<br>**741 42 Knivsta (SE)**<br>• **THURN, Tim**<br>**745 93 Enköping (SE)**<br>• **NORBERG, Gunnar**<br>**755 91 Uppsala (SE)** |
| (30) Priority: **28.04.2017 US 201715581850** | |
| (43) Date of publication of application:<br>**31.10.2018 Bulletin 2018/44** | (74) Representative: **Barker Brettell Sweden AB**<br>**Östermalmsgatan 87B**<br>**114 59 Stockholm (SE)** |
| (73) Proprietor: **C-rad Positioning AB**<br>**753 20 Uppsala (SE)** | |
| (72) Inventors:<br>• **SVENSSON, Christina**<br>**753 94 Skyttorp (SE)** | (56) References cited:<br>**EP-A2- 1 950 691      US-A1- 2009 175 406**<br>**US-A1- 2016 155 006      US-B1- 7 024 237** |

EP 3 395 408 B1

**Description**

TECHNICAL FIELD

The present embodiments generally relate to patient monitoring, and in particular to patient monitoring suitable for stereotactic radiation therapy.

BACKGROUND

Stereotactic radiation therapy (SRT) uses stereotactic technology for targeting, localization and treatment. In so-called framed SRT, a stereotactic reference system or frame is fixed to the patient and remains fixed throughout the entire radiation therapy procedure, which may last over several treatment sessions. All diagnostic examinations, such as Computed Tomography (CT) and Magnetic Resonance Imaging (MRI), generate a set of fiducial markers, which are points within the image to be localized relative to the stereotactic reference frame.

SRT can be used in connection with irradiating a target volume in different positions within the patient's body. For instance, intracranial SRT are used to treat tumors in the skull of the patient, whereas stereotactic body radiation therapy (SBRT) are used to treat tumors inside the body of the patient.

Examples of framed intracranial SRT solutions include usage of a metal frame that is invasively attached to the patient's skull. Other solutions include the use of a bite plate, to which a frame with attached markers are mounted.

US 7,024,237 discloses a face mask with passive fiducial markers that is used as a frame of reference for performing intracranial SRT. These fiducial markers can then reflect light and be detected by a camera system.

US 2011/0137102 uses a reference cage of radio-opaque material. The location of the patient's teeth is determined relative to the reference cage in order to achieve a frame of reference for performing intracranial SRT.

In frameless intracranial SRT, a head immobilization mask is used together with a localization system. For instance, Journal of Applied Clinical Medical Physics, 2010, 11(3): 26-37 discloses the usage of the BrainLAB stereotactic immobilization mask together with a kV on-board imager mounted on a Varian Trilogy machine. It was concluded that the BrainLAB mask provides a non-invasive, practical and flexible immobilization system that keeps the patient in place during treatment. However, relying on this system for patient setup might be associated with significant setup errors. Accordingly, image guidance with the kV on-board imager was needed in order to provide an independent verification technique to ensure accuracy of patient setup. It was also concluded that patient movement during the treatment may produce uncontrolled and undetected setup errors. Accordingly, the combination of stereotactic immobilization and image guidance is needed throughout the treatment sessions.

In the context of SBRT, abdominal compression equipment is typically used to immobilize and fixate the patient to be treated.

A problem with the prior art frameless SRT solutions using patient monitoring systems is that it is extremely important to discriminate between the skin of the patient and the immobilization or fixation equipment. The reason being that SRT uses a very high fraction dose and thereby the highest possible accuracy in target positioning and dose delivery is required. This means that errors in targeting and dose delivery of only about 1 mm may cause delivery of high-fraction doses in surrounding healthy tissue rather in the target volume.

Today, the discrimination between the skin of the patient and the immobilization or fixation equipment is typically done manually, in which the user selects the pixel data corresponding to the patient's skin and deselects or removes the pixel data corresponding to the immobilization or fixation equipment. This, however, requires a very skilled user with years of experience.

US 2016/0155006 discloses a device and a corresponding method for skin detection. The device comprises an illumination unit to project a predetermined illumination pattern onto a scene, an imaging unit to acquire an image of the scene and an evaluation unit to evaluate the acquired image by analyzing the imaged illumination pattern as reproduced in the image and to detect skin areas within the image and distinguish them from non-skin areas within the image based on the analysis.

There is therefore a need for an improvement in patient monitoring, and in particular in connection with frameless SRT.

SUMMARY

It is a general objective to provide an improved patient monitoring.

It is a particular objective to a patient monitoring suitable for frameless stereotactic radiation therapy.

These and other objectives are met by embodiments disclosed herein.

The present invention is defined in the independent claims. Further embodiments of the present invention are defined in the dependent claims.

A patient monitoring system comprises a threshold determining unit configured to determine a cut-off threshold value based on a signal representing a reflection response of a skin of a patient and a signal representing a reflection response of a patient covering object. The patient monitoring system also comprises a signal processing unit configured to process a reflection signal, representing a detected wave reflected from at least a portion of the patient, based on the cut-off threshold value to cut off a portion of the reflection signal corresponding to detected wave reflected from the patient covering object to get a processed reflection signal corresponding to detected wave reflected from the surface of the patient.

A patient monitoring method comprises determining a cut-off threshold value based on a signal representing a reflection response of a skin of a patient and a signal representing a reflection response of a patient covering object covering at least a portion of a surface of the patient. The method also comprises detecting a wave reflected from at least a portion of the patient and generating a reflection signal based on the detected wave. The method further comprises processing the reflection signal based on the cut-off threshold value to cut off a portion of the reflection signal corresponding to detected wave reflected from the patient covering object to get a processed reflection signal corresponding to detected wave reflected from the surface of the patient.

A computer program comprises instructions, which when executed by at least one processor, cause the at least one processor to determine a cut-off threshold value based on a signal representing a reflection response of a skin of a patient and a signal representing a reflection response of a patient covering object covering at least a portion of a surface of the patient. The at least one processor is also caused to process a reflection signal, representing a detected wave reflected from at least a portion of the patient, based on the cut-off threshold value to cut off a portion of the reflection signal corresponding to detected wave reflected from the patient covering object to get a processed reflection signal corresponding to detected wave reflected from the surface of the patient.

A related aspect of the embodiments defines a carrier comprising a computer program according to above. The carrier is one of an electronic signal, an optical signal, an electromagnetic signal, a magnetic signal, an electric signal, a radio signal, a microwave signal, or a computer-readable storage medium.

The present embodiments improve patient monitoring by cutting or cropping off a portion of the reflection signal that corresponds to a patient covering object to thereby only monitor the true surface of the patient. As a consequence, the patient monitoring of the embodiments can be used in connection with a radiation therapy machinery comprising patient covering objects that otherwise would spoil or at least make the patient monitoring more difficult.

BRIEF DESCRIPTION OF THE DRAWINGS

The embodiments, together with further objects and advantages thereof, may best be understood by making reference to the following description taken together with the accompanying drawings, in which:

Fig. 1 is a schematic illustration of a radiation gantry together with a patient monitoring system according to an embodiment;

Fig. 2 is a schematic diagram of a patient monitoring system according to an embodiment;

Fig. 3 is a schematic diagram of a patient monitoring system according to another embodiment;

Fig. 4 is a schematic diagram of a patient monitoring system according to a further embodiment;

Fig. 5 is a schematic diagram of a patient monitoring system according to yet another embodiment;

Fig. 6 is a schematic diagram of a patient monitoring system according to a further embodiment;

Fig. 7 schematically illustrates an immobilization mask used in intracranial SRT;

Fig. 8 is a schematic diagram illustrating signal amplitude along a line of a surface of a patient undergoing intracranial SRT;

Fig. 9 corresponds to the schematic diagram of Fig. 8 but applying a cut-off threshold to discriminate between patient skin and immobilization mask;

Fig. 10 corresponds to the schematic diagram of Fig. 8 but applying a cut-off threshold and a region of interest (RoI) to discriminate between patient skin and immobilization mask;

Fig. 11 is a schematic diagram illustrating an implementation embodiment of the processing part of the patient monitoring system;

Fig. 12 is a schematic diagram illustrating another implementation embodiment of the processing part of the patient monitoring system;

Fig. 13 is a schematic diagram illustrating a further implementation embodiment of the processing part of the patient monitoring system;

Fig. 14 is a schematic diagram of a computer program based implementation of an embodiment; and

Fig. 15 is a flow chart illustrating a patient monitoring method according to an embodiment.

DETAILED DESCRIPTION

Throughout the drawings, the same reference numbers are used for similar or corresponding elements.
The present embodiments generally relate to patient monitoring, and in particular to patient monitoring suitable for stereotactic radiation therapy.
The patient monitoring of the present embodiments can be used to accurately discriminate between, for instance, the skin of the patient and patient covering objects that may be present on or in the vicinity of the patient and covering at least a portion of the patient. Non-limiting, but illustrative, examples of such patient covering objects include immobilization or fixation equipment used to immobilize and fixate the patient or at least a body part of the patient to be irradiated.

In the prior art, a very skilled user with years of experience is typically needed in order to manually process image data taken by prior art patient monitoring systems in order to identify the portions of the image data that corresponds to the patient skin surface and thereby the portions of the image data that corresponds to the immobilization or fixation equipment. The latter portions, i.e., corresponding to the immobilization or fixation equipment, should preferably be deselected or cropped away to merely monitor the patient skin surface.

The reason to deselect image data corresponding to immobilization or fixation equipment is that an efficient patient monitoring should only monitor the patient skin surface. If the patient monitoring system instead would at least partly monitor the surface of the immobilization or fixation equipment thinking that the monitored surface indeed is the patient skin surface, an irradiation of the patient controlled by the patient monitoring system may miss the intended target volume and instead irradiate surrounding healthy tissue. This is in particular problematic in connection with stereotactic radiation therapy and similar radiation procedures that use a very high fraction dose, which could cause harm if deposited into healthy surrounding tissue.

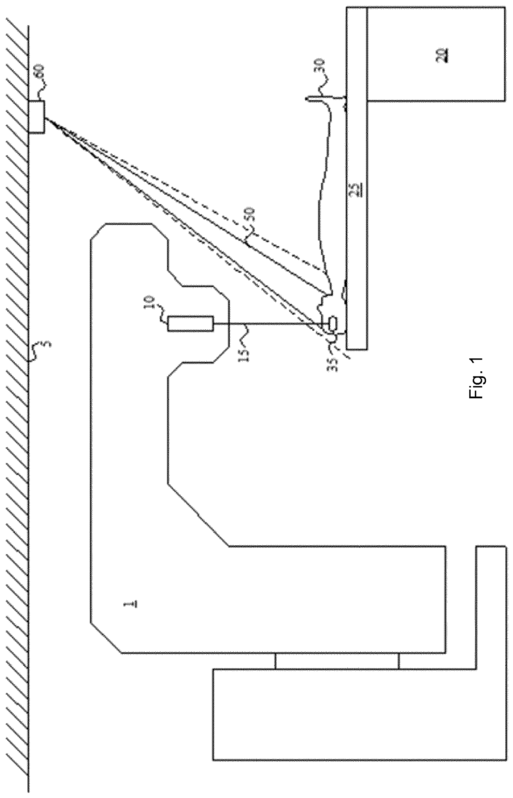

Fig. 1 is a schematic overview of a treatment room having a radiation therapy machine 1 with a radiation source 10 employed for directing radiation beams 15 into a target volume 35, such as cancerous tumor, of a patient 30 lying on a table top 25 of a patient table or couch 20. A patient monitoring system is employed to determine and control the position of the patient 30 on the patient table 20 to achieve an accurate and correct positioning of the target volume 35 relative the radiation beam 15 to meet the requirements of a previously determined treatment plan.

The patient monitoring system comprises, in an embodiment, an optional wave source configured to project a wave 50 onto at least a portion of the patient 30 on the patient table 20. In this embodiment, the patient monitoring system comprises a detector configured to detect a wave reflected from the at least a portion of the patient 30 and generate a reflection signal based on the detected wave.

The present embodiments can be used in connection with different technologies for monitoring the surface of the patient. For instance, the wave could be an electromagnetic wave that is reflected from the at least a portion of the patient and is detected by the detector to thereby generate the reflection signal. In a particular embodiment, the electromagnetic wave is visible or non-visible light. In such a case, the detector is configured to detect light reflected from the at least a portion of the patient and generate the reflection signal based on this detected light. Other examples of electromagnetic waves include microwaves and radio waves. Non-limiting examples of such microwaves and radio waves include extremely high frequency (EHF) waves, super high frequency (SHF) waves, ultra high frequency (UHF) waves, very high frequency (VHF) waves, high frequency (HF) waves, medium frequency (MF) waves, low frequency (LF) waves, very low frequency (VLF) waves, ultra low frequency (ULF) waves, super low frequency (SLF) waves and extremely low frequency (ELF) waves.

Other examples of technologies include pressure waves, such as sound or ultrasound waves, which will be further described herein.

As mentioned above, the patient monitoring system preferably comprises at least one detector configured to detect a wave reflected from at least a portion of the patient. In an optional embodiment, the patient monitoring system also comprises at least one wave source configured to project a wave onto the at least a portion of the patient.

The optional wave source could be omitted and instead use detected reflected waves originating from background or ambient sources. For instance, light detected by at least one detector could come from background or ambient light, such as lightning provided in the roofs and/or walls of the treatment room. In such a case, no dedicated light source is needed. In clear contrast, the at least one detector then detects light reflected from the at least a portion of the patient and where this light originates from the background or ambient lightning in the treatment room.

In other embodiments, at least one dedicated wave source is used to project the wave, such as electromagnetic or pressure waves, onto at least a portion of the patient. For instance, the patient monitoring system could comprises a light source configured to project light 50 onto at least a portion of the patient 30. In a particular embodiment, the light source is configured to project a 2- or 3-dimensional (2D or 3D) light pattern 50 onto a surface of the patient 30. In this embodiment, the patient monitoring system also comprises at least one detector, such as at least one light detector, configured to detect light 50 projected onto the at least a portion of the patient 30 and generate a reflection signal based on the detected light.

In Fig. 1, the patient 30 is lying on a patient table or couch 20 during the irradiation session. The embodiments are, however, not limited thereto. In specific forms of irradiation, the patient 30 could instead be sitting or indeed standing.

The patient monitoring system could comprise a single detector and optionally a single wave source. In other embodiments, the patient monitoring system comprises multiple, i.e., at least two, detectors and optionally a single wave source, a single detector and multiple waves sources or multiple detectors and multiple wave sources.

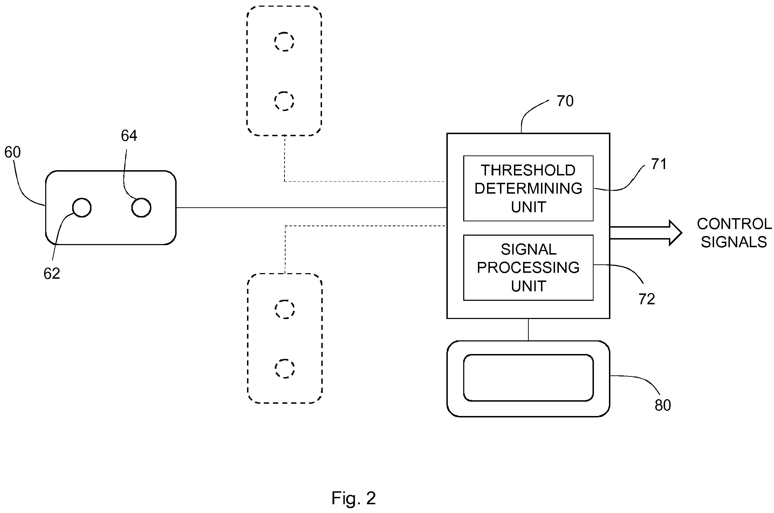

For instance, a wave source 62 and a detector 64 may be arranged together in a unit or device 60 as is more clearly shown in Fig. 2. Such a unit 60 may then, for instance, be arranged on the roof 5 in the treatment room, on a wall in the treatment room, attached to the gantry of the radiation therapy machine 1 and/or elsewhere arranged in connection with the radiation therapy machine 1 in order to project a wave 50 onto the patient 30 and detect the reflected wave. Fig. 2 also indicates the presence of multiple, such as three, units 60 with a respective wave source 62 and detector 64 arranged in connection with the radiation therapy machine 1.

An example of a light source and light detector that could be used according to the embodiments is disclosed in U.S. patent no. 8,235,530. Other examples include patient positioning equipment by C-RAD AB, such as Catalyst, and in particular Catalyst HD™.

Catalyst HD™ is highly compatible with intracranial SRT using high doses since it achieves high-precision patient positioning and intra-fraction motion management. During treatment, patient breathing, as well as movement of the tumor or normal tissue can impact the clinical result by missing targets or causing extra dose distribution to normal tissue.

Catalyst HD™ offers a complete Surface Image Guided Radiation Therapy (SIGRT) solution on SRT and SRS for online patient tracking before and during treatment delivery, thus ensuring the best possible treatment outcome without non-prescribed doses. In the treatment room, Catalyst HD™ provides the following benefits compared to other SIGRT solutions:

- submillimeter accuracy surface image guided SRT solution;
- Catalyst HD™ is integrated into the stereotactic treatment workflow, from setup and positioning to intra-fraction motion monitoring to respiratory gating treatment with submillimeter accuracy for non-coplanar treatment;
- during treatment, the patient is constantly under surveillances by Catalyst HD™;
- correct any posture or positioning errors immediately;
- auto couch correction in set-up;
- any patient movements over tolerance immediately trigger beam hold; and
- respiratory gating.

The present embodiments are, however, not limited to the above mentioned light-based patient monitoring solutions but can be used in connection with any light source configured to project light onto at least a portion of the patient on the patient table and any light detector configured to detect light projected onto the at least a portion of the patient and generate a light signal based on the detected light.

Non-limiting, but illustrative examples, of radar based technology for the wave source and detector include the usage of ultra wideband (UWB) impulse radar and electromagnetic time of flight detector. Such UWB based wave sources and detectors are available on the market by XeThru, such as XeThru X2M200 Respiration Module. Other examples using UWB and radar technology are described in U.S. patent nos. 6,919,838; 9,468,395; and U.S. patent application nos. 2016/0081618; 2010/0185102; 2015/0320342.

Equipment for monitoring patient surfaces using pressure waves, such as sound or ultrasound waves, are well known in the art. Any such pressure wave source and pressure wave detector could be used according to the embodiments.

According to the embodiments, a cut-off threshold value is determined based on a signal representing a reflection response of a skin of a patient and a signal representing a reflection response of a patient covering object. The type of reflection response depends on the type of technology, i.e., the type of wave reflected from patient. For instance, in the case of detection of reflected light, the reflection response is preferably the color or tone of the skin of the patient and the color or tone of the patient covering object. In the case of other electromagnetic waves and pressure waves, the reflection response could be surface structures or surface composition as determined based on the detected reflected electromagnetic or pressure waves. Also, different materials have different absorption and reflection properties for different types of waves, such as electromagnetic waves of different wavelengths and frequencies. Hence, reflection response as used herein could also be absorption or reflection properties or characteristics.

Fig. 2 is a schematic diagram of a patient monitoring system 70 according to an embodiment. The patient monitoring system 70 comprises a threshold determining unit 71 and a signal processing unit 72. The threshold determining unit 71 is configured to determine a cut-off threshold value based on a signal representing a reflection response of a skin of a patient and a signal representing a reflection response of a patient covering object. The patient covering object covers at least a portion of a surface of the patient. The signal processing unit 72 is configured to process a reflection signal representing a detected wave reflected from at least a portion of the patient. The signal processing unit 72 is configured to process the reflection signal based on the cut-off threshold value to cut off a portion of the reflection signal corresponding to detected wave reflected from the patient covering object to get a processed reflection signal corresponding to detected wave reflected from the surface of the patient.

In an embodiment, the signal processing unit 72 is configured to process the reflection signal, representing a detected electromagnetic wave reflected from the at least a portion of the patient, based on the cut-off threshold value to cut off a portion of the reflection signal corresponding to detected electromagnetic wave from the patient covering object to get the processed reflection signal corresponding to detected electromagnetic wave reflected from the surface of the patient.

In a particular embodiment, the threshold determining unit 71 is configured to determine a cut-off threshold value based on a signal representing a color or tone of a skin of a patient and a signal representing a color or tone of a patient covering object. The patient covering object covers at least a portion of a surface of the patient. The signal processing unit 72 is configured to process a reflection signal representing detected light reflected from, and optionally projected onto, at least a portion of the patient on the patient table. The signal processing unit 72 is configured to process the reflection signal based on the cut-off threshold value to cut off a portion of the reflection signal corresponding to detected light reflected from the patient covering object to get a processed reflection signal corresponding to detected light reflected from the surface of the patient.

In another embodiment, the signal processing unit is configured to process the reflection signal, representing a detected pressure, such as sound or ultrasound, wave reflected from the at least a portion of the patient, based on the cut-off threshold value to cut off a portion of the reflection signal corresponding to detected pressure wave from the patient covering object to get the processed reflection signal corresponding to detected pressure wave reflected from the surface of the patient.

Thus, according to the embodiments, signals representing the reflection response, such as the color or tone, of the skin of the patient and of the patient covering object are used to determine the value of a cut-off threshold. This means that the value of this cut-off threshold is determined based on the reflection response, such as the color or tone, of the patient's skin and the reflection response, such as the color or tone, of the patient covering object. The cut-off threshold value as determined by the threshold determining unit 71 is then used by the signal processing unit 72 in the processing of the reflection signal. Thus, the signal processing unit 72 uses the cut-off threshold value to cut off or crop the reflection signal to thereby get the processed reflection signal. This processed reflection signal corresponds to the detected wave reflected from the surface of the patient, whereas the detected wave reflected from the patient covering object has been cut or cropped off by means of the cut-off threshold.

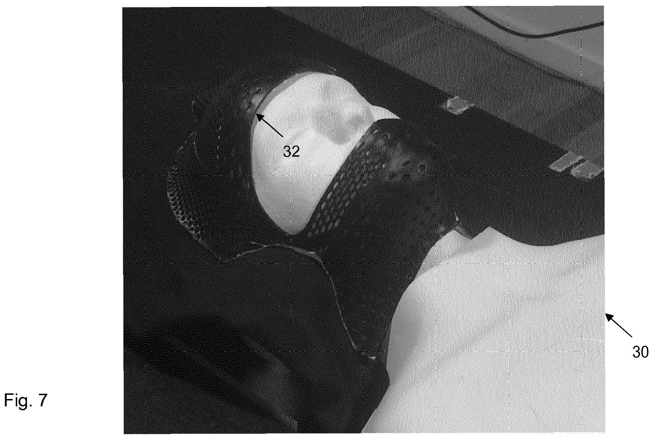

This situation is schematically illustrated in Figs. 7 to 9. Fig. 7 illustrates an immobilization or fixation mask 32 for intracranial SRT as an illustrative, but non-limiting, example of a patient covering object 32 covering at least a portion of a surface of the patient 30, in this case covering a portion of the surface of the patient's skull.

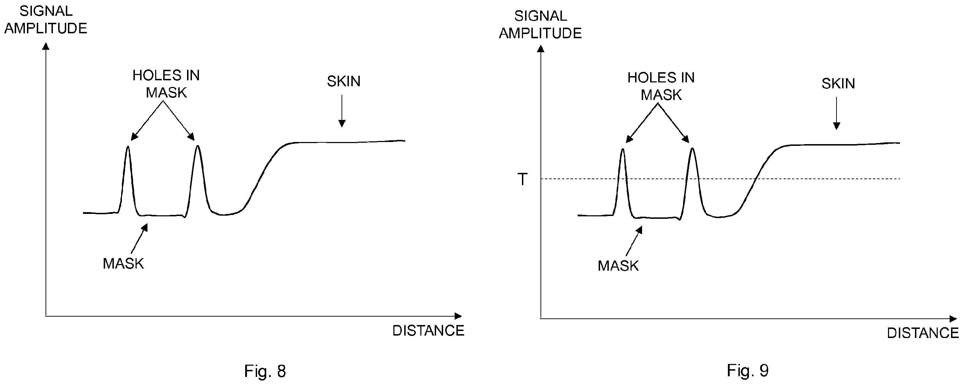

Fig. 8 is a schematic diagram illustrating signal amplitude of the reflection signal along a line of a surface of a patient undergoing intracranial SRT. The left part of the curve corresponds to the reflection signal from the immobilization mask, with peaks in the signal corresponding to reflected wave detected from holes in the immobilization mask. The right part of the curve corresponds to the reflection signal from the skin of the patient 30, i.e., from the large opening in the immobilization mask 32 aligned with the lower forehead, the eyes, the nose and the cheeks of the patient 30 as seen in Fig. 7.

If the patient monitoring would have been based on the reflection signal as illustrated in Fig. 8, the surface of not only patient's skin but also the surface of the immobilization mask would be monitored. This could lead to significant problems in controlling the irradiation and targeting a target volume within the patient's skull based on the monitored surface. Thus, an accurate patient monitoring should merely be based on the surface corresponding to the skin of the patient and not the surface of the immobilization mask.

Fig. 9 illustrates the schematic diagram of Fig. 8 but with the cut-off threshold value (T) as determined according to the embodiments. In this case, the signal processing unit will cut off or crop the portion of the reflection signal that, in this example, falls below the cut-off threshold value, i.e., the portion of the signal having a signal amplitude less than the threshold value. This cut off effectively removes the portion of the reflection signal corresponding to detected wave reflected from the immobilization mask. Accordingly, the remaining portion of the reflection signal, i.e., having a signal amplitude, in this example, larger than the cut-off threshold value, constitutes the processed reflection signal that corresponds to detected wave reflected from the surface of the patient, i.e., from the small holes in the immobilization mask and from the larger opening in the immobilization mask.

As mentioned in the foregoing, in an embodiment, the patient monitoring system comprises a detector configured to detect the wave reflected from the at least a portion of the patient and generate the reflection signal based on the detected wave.

In a particular embodiment, the patient monitoring system also comprises a wave source configured to project a wave onto the at least a portion of the patient.

In the following, various embodiments will be described in more detail and in particular to the case of having color or tone as the reflection response and detection of light reflected from, and optionally projected onto, at least a portion of the patient. These embodiments can, however, also be applied to other types of reflection responses and other types of waves than light.

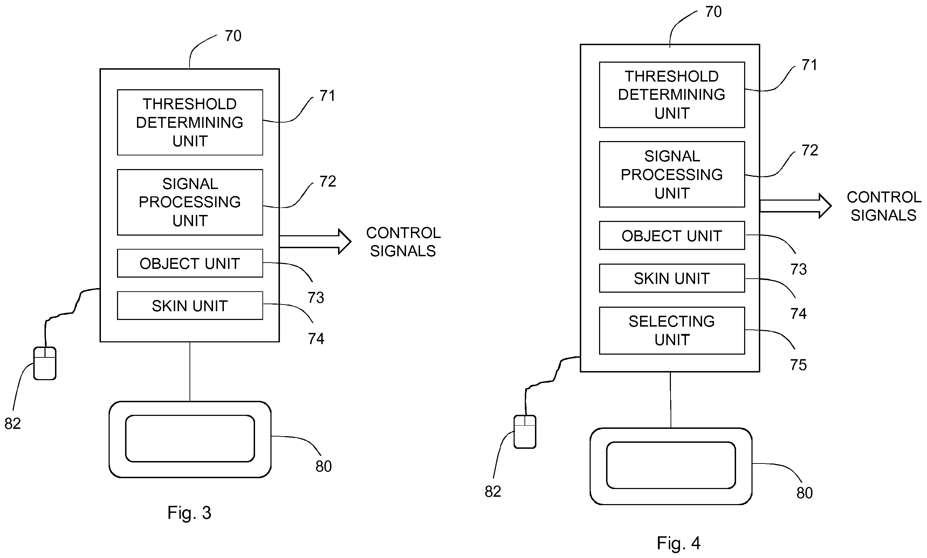

Fig. 3 is a schematic block diagram of a patient monitoring system 70 according to another embodiment. In this embodiment, the patient monitoring system 70 comprises an object unit 73 in addition to the threshold determining unit 71 and the signal processing unit 72. The object unit 73 is configured to generate the signal representing the reflection response, such as the color or tone, of the patient covering object based on activation of a user input 80, 82.

In a particular embodiment, the object unit 73 is configured to generate the signal representing the reflection response, such as the color or tone, of the patient covering object as representing a reflection response, such as a color or tone, selected from a set of multiple predefined reflection responses, such as colors or tones, based on activation of the user input 80, 82.

Thus, there is a set of multiple predefined reflection responses, such as colors or tones, to select among using a user input 80, 82. The user input could, for instance, be a touch-sensitive screen 80, a mouse 82, a keyboard or some other user input. In such a case, the user selects one of the multiple predefined reflection responses, such as colors or tones, using the user input 80, 82, which causes the object unit 73 to generate the signal representing the selected reflection response, such as color or tone, of the patient covering object.

For instance, a screen or display 80 could be connected to or included in the patient monitoring system 70. In such a case, the screen 80 could be configured to display the multiple predefined reflection responses, such as colors or tones, to select among. The user then selects one of these multiple predefined reflection responses, such as colors or tones, that best matches the actual reflection response, such as color or tone, of the patient covering object. For instance, if the patient covering object is very light the user could select a white color from the set of multiple predefined color or tones using the user input 80, 82. Correspondingly, if the patient covering object is very dark, the user could select a black color.

The object unit 73 then forwards the generated signal representing the selected reflection response, such as color or tone, of the patient covering object to the threshold determining unit 71 to be used therein to determine the value of the cut-off threshold.

In an alternative, or additional, embodiment, the patient monitoring system 70 comprises a skin unit 74 in addition to the threshold determining unit 71 and the signal processing unit 72, and optionally in addition to the object unit 73. The skin unit 74 is configured to generate the signal representing the reflection response, such as the color or tone, of the skin of the patient based on activation of a user input 80, 82.

In a particular embodiment, the skin unit 74 is configured to generate the signal representing the reflection response, such as the color or tone, of the skin of the patient as representing a reflection response, such as a color or tone, selected from a set of multiple predefined reflection responses, such as colors or tones, based on activation of the user input 80, 82.

Thus, there is a set of multiple predefined reflection responses, such as colors or tones, to select among using the user input 80, 82. The screen 80 could be configured to display the multiple predefined reflection responses, such as colors or tones, to select among. In such a case, the user selects one of the multiple predefined reflection responses, such as colors or tones, using the user input 80, 82, which causes the skin unit 74 to generate the signal representing the selected reflection response, such as color or tone, of the skin of the patient.

For instance, the set could include a dark skin tone, a medium skin tone and a pale skin tone. The user then selects one of these skin tones that best matches the tone of the skin of the current patient using the user input 80, 82 to thereby cause the skin unit 74 to generate the signal representing the color or tone of the skin of the patient. The skin unit 74 outputs the generated signal to the threshold determining unit 71 to be used therein to determine the value of the cut-off threshold.

In another embodiment, the skin unit 74 is configured to generate the signal representing the reflection response, such as the color or tone, of the skin of the patient based on a reflection signal generated by the detector and representing detected wave, such as light, reflected from at least a portion of the patient.

Thus, in this embodiment, the reflection response, such as the color or tone, of the skin is determined based on detection of a wave, such as light, optionally projected onto and reflected from the skin of the patient rather based on a manual selection by the user. In a particular embodiment, the detector is configured to detect the wave, such as light, reflected from the at least a portion of the patient prior to covering the at least a portion of the patient by the patient covering object and to generate the reflection signal based on the detected reflected wave, such as light. In this particular embodiment, the wave, such as the light, is preferably projected from a wave source, such as a light source, onto the patient prior to arranging the patient covering object onto or in the vicinity of the patient. Accordingly, reflected wave, such as light, detected by the detector thereby mainly corresponds to the wave, such as light, projected onto the surface of the patient. This detected reflected wave, such as light, can then be used by the skin unit 74 to generate the signal representing the reflection response, such as the color or tone, of the skin of the patient.

In an alternative, or additional, embodiment, the object unit 73 is configured to generate the signal representing the reflection response, such as the color or tone, of the patient covering object based on a reflection signal generated by the detector and representing detected wave, such as light, reflected from at least a portion of the patient covering object.

Thus, in this embodiment, the reflection response, such as the color or tone, of the patient covering object is determined based on detection of a wave, such as light, optionally projected onto and reflected from the patient covering object rather based on a manual selection by the user. In a particular embodiment, the detector is configured to detect the wave, such as light, reflected from the at least a portion of the patient covering object. This could, for instance, be performed by detecting the wave, such as light, reflected from the patient covering object before positioning a patient on the patient table. In this particular embodiment, the wave, such as the light, is preferably projected from a wave source, such as a light source, onto the patient covering object. Accordingly, reflected wave, such as light, detected by the detector thereby mainly corresponds to the wave, such as light, projected onto the surface of the patient covering object. This detected reflected wave, such as light, can then be used by the object unit 73 to generate the signal representing the reflection response, such as the color or tone, of the patient covering object.

In an embodiment, the patient monitoring system 70 comprises any of the embodiments of the object unit 73 described above. In another embodiment, the patient monitoring system 70 comprises any of the embodiments of the skin unit 74 described above. In a further embodiment, the patient monitoring system 70 comprises any of the embodiments of the object unit 73 and the skin unit 74 described above as schematically illustrated in Fig. 3.

In an alternative, or additional embodiment, see Fig. 4, the patient monitoring system 70 comprises a screen 80 configured to display an image generated based on the reflection signal generated by the detector and representing detected wave, such as light, optionally projected onto and reflected from the at least a portion of the patient, and optionally prior to covering at least a portion of the patient by the patient covering object. The patient monitoring system 70 also comprises a selecting unit 75. In an embodiment, the selecting unit 75 is configured to generate a selection signal representing a selected portion of the image displayed on the screen 80. The selecting unit 75 generates this selection signal based on activation of a user input 80, 82. In this embodiment, the skin unit 74 is configured to generate the signal representing the reflection response, such as the color or tone, of the skin of the patient based on a reflection response, such as color or tone, of the portion of the image selected based on the selection signal.

Hence in an embodiment, the light source projects light onto the patient and the detector detects this projected light and generates the reflection signal. The reflection signal is processed by the patient monitoring system 70 to generate an image of at least a portion of the patient based on the reflection signal. This image is displayed on the screen 80. The user then uses the user input 80, 82 to select a portion of the image that the user considers corresponding to the skin of the patient. This can, for instance, be performed by moving a cursor over the image until the cursor is on a portion of the image that the user considers to be the skin of the patient. The user could then click on a mouse 82, a key, or push onto the touch sensitive screen 80. The selecting unit 75 thereby generates a selection signal representing this selected portion, such as the coordinates of the selected point within the image. In such a case, the skin unit 74 generates a signal representing the color or tone of the selected portion, such as the color of the image at the coordinates represented by the selection signal.

In an alternative, or additional embodiment, the patient monitoring system 70 comprises a screen 80 configured to display an image generated based on the reflection signal generated by the detector and representing detected wave, such as light, optionally projected onto, and reflected from the at least a portion of the patient covering object. The patient monitoring system 70 also comprises a selecting unit 75. In an embodiment, the selecting unit 75 is configured to generate a selection signal representing a selected portion of the image displayed on the screen 80. The selecting unit 75 generates this selection signal based on activation of a user input 80, 82. In this embodiment, the object unit 73 is configured to generate the signal representing the reflection response, such as the color or tone, of the patient covering object based on a reflection response, such as a color or tone, of the portion of the image selected based on the selection signal.

Hence in an embodiment, a light source projects light onto at least a portion of the patient covering object and the detector detects this projected light and generates the reflection signal. The reflection signal is processed by the patient monitoring system 70 to generate an image of at least a portion of the patient covering object based on the light signal. This image is displayed on the screen 80. The user then uses the user input 80, 82 to select a portion of the image that the user considers corresponding to the patient covering object. This can, for instance, be performed by moving a cursor over the image until the cursor is on a portion of the image that the user considers to be the patient covering object. The user could then click on a mouse 82, a key, or push onto the touch sensitive screen 80. The selecting unit 75 thereby generates a selection signal representing this selected portion, such as the coordinates of the selected point within the image. In such a case, the object unit 73 generates a signal representing the color or tone of the selected portion, such as the color of the image at the coordinates represented by the selection signal.

In an embodiment, the skin unit 74 is configured to identify, in an image generated based on the reflection signal, a portion of the image corresponding to the skin of the patient based on a reference image of the patient and/or of the patient covering object. The skin unit 74 is also configured to generate the signal representing the reflection response, such as the color or tone, of the skin of the patient based on a reflection response, such as a color or tone, of the portion of the image identified based on the reference image.

In an embodiment, the object unit 73 is configured to identify, in an image generated based on the reflection signal, a portion of the image corresponding to the patient covering object based on a reference image of the patient and/or the patient covering object. The object unit 73 is also configured to generate the signal representing the reflection response, such as the color or tone, of the patient covering object based on a reflection response, such as a color or tone, of the portion of the image identified based on the reference image.

In these embodiments, a portion of the image corresponding to the skin of the patient is identified based on a reference image of the patient, such as skin of the patient, and/or of the patient covering object, and/or a portion of the image corresponding to the patient covering object is identified based on a reference image of the patient and/or of the patient covering object. The reference image(s) could be previously generated image(s) of (the skin of) the patient and/or of the patient covering object, possibly taken in another radiation machine, such as CT or MRI, than the current radiation machine and stored in a memory. In such a case, the skin unit 74 and/or the object unit 73 retrieves the reference image(s) from the memory and uses it/them to identify the portion of the current image that corresponds to the skin of the patient and/or the patient covering object.

The skin unit 74 and/or the object unit 73 could perform a pattern or image recognition or matching process in order to identify the portion(s) of the current image that matches with the reference images. It is then possible to have the skin unit 74 identifying a first portion of the image using a reference image of skin of the patient and the object unit 73 identifying a second portion of the image using a reference image of the patient covering object.

In either case, the reflection response, such as the color or tone, of the skin of the patient is then determined based on the reflection response, such as a color or tone, of the portion of the image identified by the skin unit 74 and/or the reflection response, such as the color or tone, of the patient covering object is determined based on the reflection response, such as a color or tone, of the portion of the image identified by the object unit 73.

Thus, in embodiments, the patient monitoring system 70 could automatically identify a point or area of the image that corresponds to the patient's skin and/or a point or area of the image that corresponds to the patient covering object. This identification could be done automatically, such as based on a reference image as mentioned above.

Alternatively, the identification can be done based on an estimation of the size and/or shape of the patient's skin, such as corresponding to shape and size of the large opening in an immobilization mask 32, see Fig. 7, and/or an estimation of the size and/or shape of the immobilization mask 32. This identification does not need to perfectly identify the exact area corresponding to the skin and the exact area corresponding to the patient covering object, such as immobilization mask. For instance, it can be assumed that a central point or area in the image corresponds to the skin area, whereas a peripheral point or area in the image corresponds to the mask area. In this latter case, it is assumed that the detector is focusing on the patient's skin, such as on the large opening in the immobilization mask. Hence, the central portion of the resulting image would correspond to the skin within this large opening, whereas surrounding peripheral portion of the image corresponds to the immobilization mask.

Hence, in an embodiment, the skin unit 74 is configured to generate the signal representing the reflection response, such as the color or tone, of the skin of the patient based on a reflection signal generated by the detector and representing detected wave, such as light, reflected from a central portion of a field of view of the detector. Correspondingly, in an embodiment, the object unit 73 is configured to generate the signal representing the reflection response, such as the color or tone, of the patient covering object based on a reflection signal generated by the detector and representing detected wave, such as light, reflected from a peripheral portion of a field of view of the detector.

As an alternative to this automatic identification based on i) reference image of the skin of the patient, ii) reference image of the patient covering object, iii) shape and/or size of the patient covering object, iv) (peripheral) position of the patient covering object, v) shape and/or size of the skin area, and/or vi) (central) position of the skin area, the user could mark a point or area in the image that the user regards as corresponding to the patient's skin and/or mark a point or area in the image that the user regards as corresponding to the patient covering object.

In either case, the signal representing the reflection response, such as the color or tone, of the patient's skin and the signal representing the reflection response, such as the color or tone, of the patient covering object are then generated based on the image data of the identified or marked point or area.

In an embodiment, the patient monitoring system 70 comprises any of the embodiments of the object unit 73 described above in connection with Fig. 4. In another embodiment, the patient monitoring system 70 comprises any of the embodiments of the skin unit 74 described above in connection with Fig. 4. In a further embodiment, the patient monitoring system comprises any of the embodiments of the object unit 73 and the skin unit 74 described above as schematically illustrated in Fig. 4.

The embodiments of the object unit 73 and the skin unit 74 in Figs. 3 and 4 could be combined. For instance, the patient monitoring system 70 could comprise an object unit 73 configured to generate the signal representing the reflection response, such as the color or tone, of the patient covering object based on activation of a user input 80, 82 and a skin unit 74 configured to generate the reflection response, such as the color or tone, of the skin of the patient based on a reflection signal generated by the detector. Alternatively, the patient monitoring system 70 could comprise an object unit 73 configured to generate the signal representing the reflection response, such as the color or tone, of the patient covering object based on a reflection signal generated by the detector and a skin unit 74 configured to generate the signal generating the reflection response, such as the color or tone, of the skin of the patient based on activation of a user input 80, 82. These embodiments thereby combine one of the object unit 73 and the skin unit 74 from Fig. 3 with the other of the object unit 73 and the skin unit 74 from Fig. 4.

Thus, the patient monitoring system 70 could include an object unit 73 according to any of the embodiments described in connection with Fig. 3 and a skin unit 74 according to any of the embodiments described in connection with Fig. 4. Alternatively, the patient monitoring system 70 could include an object unit 73 according to any of the embodiments described in connection with Fig. 4 and a skin unit 74 according to any of the embodiments described in connection with Fig. 3.

In either case, the threshold determining unit 71 is configured to determine the cut-off threshold value $T$ based on the signal representing the reflection response of the skin of the patient, $RR_{skin}$, and the signal representing the reflection response of the patient covering object, $RR_{PCO}$. In an embodiment, the threshold determining unit 71 is configured to determine the cut-off threshold value as a function $f(\ )$ of the reflection responses, i.e., $T = f(RR_{skin}, RR_{PCO})$. Various functions could be used and are within the scope of the embodiments. For instance, the cut-off threshold value could be calculated as the average of the (mean) reflection responses, such as $T = \frac{RR_{skin} + RR_{PCO}}{2}$. More generally, the function could be a weighted combination of the (mean) reflection responses, such as $T = \alpha RR_{skin} + (1 - \alpha)RR_{PCO}$ for some value $\alpha \in (0,1)$.

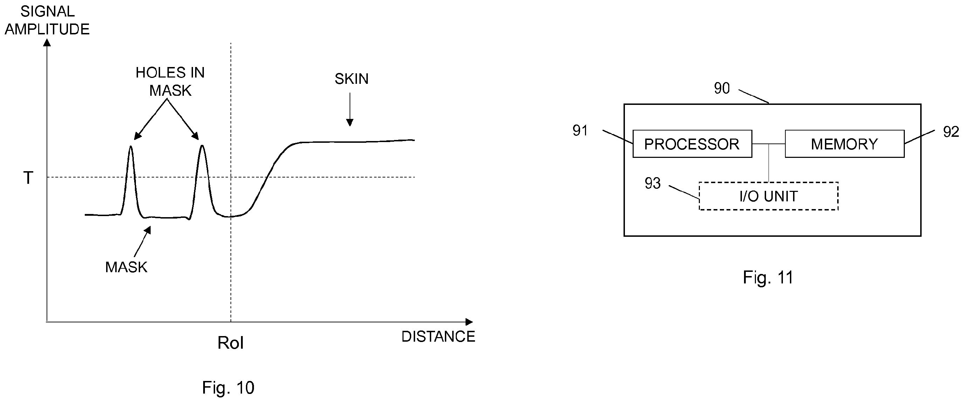

In an embodiment, the signal processing unit 72 processes the reflection signal from the detector not only based on the cut-off threshold value determined by the threshold determining unit 71 but also based on a region of interest (RoI). This is schematically illustrated in Fig. 10. In such a case, this region of interest defines a region of the patient comprising the surface of the patient.

For instance, the region of interest can be used to define a region, area or portion of the image displayed on the screen 80 that comprises the patient's skin. This region of interest could roughly correspond to the large opening in the immobilization mask 32 shown in Fig. 7 or at least a portion thereof. The region of interest limitation can be used to remove signals corresponding to the comparatively small parts of the skin from the holes in the immobilization mask. In a typical embodiment, the region of interest is defined by the user using a user input 80, 82.

Hence, the user could use the user input 80, 82 to delimit a relevant area within the displayed image and where this delimited area corresponds to the region of interest. For instance, a rectangular area could be indicated by the user input 80, 82 to roughly correspond to the large opening in the immobilization mask. In such a case, only the portion of the reflection signal from this region of interest and left following cropping with the cut-off threshold value will remain as the processed reflection signal. This corresponds to the upper right quadrant in Fig. 10.

Instead of manually defining the region of interest, a pattern recognition or image matching process could be used employing reference images of at least a portion of the patient and/or of the patient covering object. Such reference image(s) could then be matched and aligned with the current image and thereby be used to identify a portion of the current image that corresponds to the region of interest.

Hence, in an embodiment, the signal processing unit 72 is configured to process the reflection signal based on the cut-off threshold value and a signal representing a region of interest to cut off a portion of the reflection signal corresponding to detected wave, such as light, optionally projected onto, and reflected from the patient covering object to get the processed reflection signal corresponding to detected wave, such as light, optionally projected onto, and reflected from the surface of the patient. In this embodiment, the signal representing the region of interest defines a region of the patient comprising the surface of the patient.

The patient monitoring system of the embodiments designed to accurately monitor the skin surface of a patient can be used for various purposes in connection with a radiation therapy machine. For instance, the patient monitoring system can be used during patient set-up and positioning. The patient monitoring system can thereby be used to correctly position the patient, such as on the patient table and relative to the radiation therapy machine and the radiation source. Any positioning or posture errors can be detected by the patient monitoring system.

The patient monitoring system can also be used for intra-fraction motion detection, i.e., detecting any changes in position or posture of the patient throughout the treatment to permit accurate delivery of the irradiation dose within the target volume.

The patient monitoring system may also be used for respiratory gating solutions, such as for both free breathing and deep inspiration breath hold (DIBH).

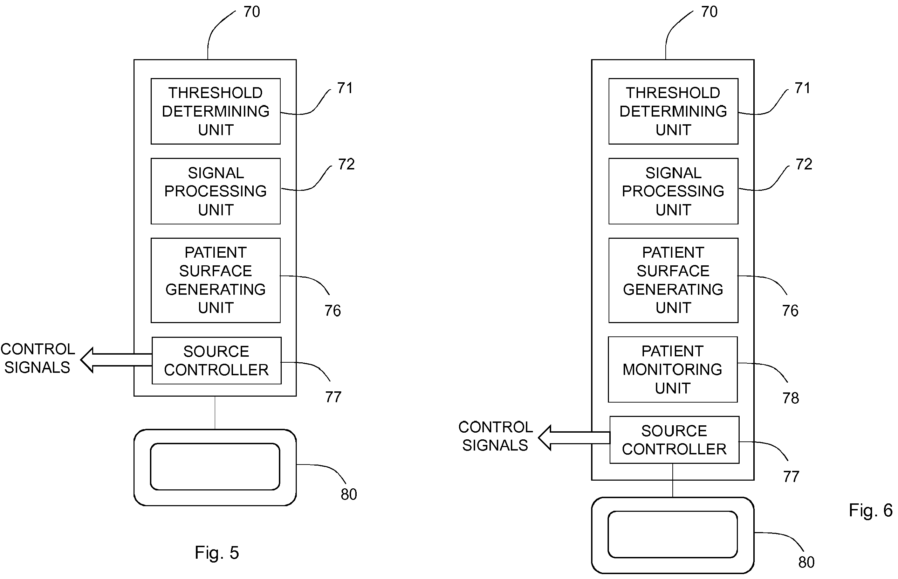

Thus, in an embodiment and as shown in Fig. 5, the patient monitoring system 70 comprises a patient surface generating unit 76 configured to generate a signal representing at least a portion of the surface of the patient based on the processed reflection signal. The signal generated by the patient surface generating unit 76 is generally more accurate by being based on the processed reflection signal from the signal processing unit 72 rather than being based on the (raw) reflection signal from the detector. The increase in accuracy is achieved by processing the reflection signal as disclosed herein based on the determined cut-off threshold value and optionally also based on the RoI representing signal. This signal processing is performed by the signal processing unit 72 in order to remove the portions of the reflection signal that corresponds to the surface of the patient covering object to thereby only or at least mainly monitor the true skin surface of the patient.

In an embodiment, the patient monitoring system 70 also comprises a source controller 77 connected to the patient surface generating unit 76 and a radiation source configured to irradiate a target volume within the patient on the patient table. The source controller 77 is configured to control irradiation from the radiation source based on the signal representing the at least a portion of the surface of the patient.

For instance, the source controller 77 could control the radiation source to only irradiate the target volume within the patient in synchrony with a selected phase of the respiratory cycle, such as in synchrony with inspiration or expiration. This gating of dose delivery in synchrony with the respiratory cycle reduces the risk that the target volume has moved between dose delivery occasions. For instance, if the target volume is moved slightly within the patient body between inspiration and expiration, a more accurate dose delivery is achieved by only irradiating the target volume in synchrony with a selected phase of the respiratory cycle. In such a case, the patient surface generating unit 76 preferably continuously or at least with a sufficiently high sample rate monitors the skin surface of the patient by generating signals representing the current position of the surface of the skin of the patient based on the processed reflection signal. The source controller 77 could then identify, from the signal output by the patient surface generating unit 76, when the patient is within the selected phase of the respiratory cycle based on the monitored skin surface.

Fig. 6 is a schematic block diagram of yet another embodiment of the patient monitoring system 70. In this embodiment, the patient monitoring system 70 comprises a patient monitoring unit 78 configured to generate a difference signal based on a comparison between the signal representing the at least a portion of the surface of the patient and a reference signal representing a reference surface. Thus, in this embodiment, the patient monitoring unit 78 is configured to generate the difference signal based on the signal output from the patient surface generating unit 76 and the reference signal. The difference signal can then be used to, for instance, for accurate patient setup and positioning and/or motion detection.

In the former case, the reference signal represents a reference surface according to the treatment set-up plan and preferable defines a correct position and posture of the patient on the patient table. The patient monitoring system can then be used to accurately monitor the surface of the patient to correctly position the patient on the patient table. Thus, the patient is repositioned until the difference signal represents a difference between actual patient position and posture, as represented by the signal from the patient surface generating unit 76 and generated based on the processed light signal, and the target position and posture, as represented by the reference signal, that is smaller than a defined value.

In the latter case, the patient monitoring system 70 may optionally comprises a source controller 77 connected to the patient monitoring unit 78 and to a radiation source configured to irradiate a target volume within the patient. In this embodiment, the source controller 77 is configured to control irradiation from the radiation source based on the difference signal from the patient monitoring unit 78.

For instance, the source controller 77 could be configured to interrupt irradiation from the radiation source if the difference signal indicates an unacceptably large difference between the actual position and posture of the skin surface of the patient and the reference surface. This means that the source controller 77 stops dose delivery if the patient has moved to thereby prevent dose delivery outside of the target volume.

The embodiments of the patient monitoring system described above and illustrated in Figs. 2 to 6 may be combined. For instance, the embodiments shown in Figs. 5 and 6 may comprise an object unit, a skin unit and/or a selecting unit as illustrated in Figs. 3 and 4.

In some applications, there may be a set of available patient covering objects to select among and where the different patient covering objects have different reflection responses, such as colors or tones. For instance, an immobilization mask used in frameless stereotactic radiation therapy may be available in a dark, such as black, color and in a light, such as white, color. In such a case, the user preferably selects a patient covering object having a color with a large contrast to the color of the skin of the patient. For instance, a dark colored immobilization mask is preferably used for pale toned patients, whereas the light colored immobilization mask is preferably used for dark toned patients. This selection of the colored patient covering object maximizes or at least increases the contrast between the color or tone of the patient's skin and the color or tone of the patient covering object. This will in turn lead to a large difference in signal amplitudes between the portion of the signal corresponding to the patient covering object (see mask in Figs. 8-10) and the portion of the signal corresponding to the patient (see skin in Figs. 8-10).

The above described embodiments generate a processed signal based on the reflection response, such as color or tone, of the skin of the patient and the reflection response, such as color or tone, of the patient covering object. The embodiments may also be applied to situations where multiple, possibly different, patient covering objects can be present and covering at least different portions of the patient. In such a case, the value of the cut-off threshold is preferably determined based on the reflection response, such as the color or tone, of the patient's skin and the reflection responses, such as the colors or tones, of these multiple patient covering objects, e.g., $T = f(RR_{skin}, RR_{PCO1}, RR_{PCO2}, ...)$.

Alternatively, multiple cut-off threshold values can be determined. For instance, assume that a first patient covering object is covering at least a first portion of the surface of the patient, whereas a second patient covering object is covering at least a second portion of the surface of the patient. The first and second portions are at least partly spatially separated from each other. In such a case, a first cut-off threshold value could be determined based on a signal representing the reflection response, such as the color or tone, of the skin of the patient and a signal representing the reflection response, such as the color or tone, of the first patient covering object, e.g., $T_1 = f(RR_{skin}, RR_{PCO1})$. Correspondingly, a second cut-off threshold value is determined based on the signal representing the reflection response, such as the color or tone, of the skin of the patient and a signal representing the reflection response, such as the color or tone, of the second patient covering object, e.g., $T_2 = f(RR_{skin}, RR_{PCO2})$. In such a case, the first cut-off threshold value could be used to cut off a first portion of the reflection signal corresponding to detected wave, such as light, reflected from the first patient covering object and the second cut-off threshold is used to cut off a second portion of the reflection signal corresponding to detected wave, such as light, reflected from the second patient covering object to get the processed reflection signal.

Illustrative, but non-limiting, examples of patient covering objects include immobilization masks for frameless intracranial stereotactic radiation therapy; immobilization equipment for stereotactic body radiation therapy, such as abdominal compression equipment, e.g., abdominal compression belts and abdominal compression masks; blankets and covers; radio frequency (RF) coils in magnetic resonance (MR) applications; life-supporting equipment; a bolous; etc. Thus, the patient covering objects could be any equipment or devices present on or in connection with the patient and at least partly covering a skin surface of the patient as seen by the detector(s).

In a particular embodiment, the patient covering object is an immobilization mask, such as for frameless intracranial stereotactic radiation therapy. The immobilization mask is configured to fixate or immobilize a head of the patient. In this particular embodiment, the signal processing unit is configured to process the reflection signal based on the cut-off threshold value to cut off a portion of the reflection signal corresponding to detected wave, such as light, optionally projected onto, and reflected from the immobilization mask to get a processed reflection signal corresponding to detected wave, such as light, optionally projected onto, and reflected from a surface of the head of the patient.

In another particular embodiment, the patient covering object is an abdominal compression equipment, such as for stereotactic body radiation therapy. The abdominal compression equipment is configured to fixate or immobilize an abdominal of the patient. In this particular embodiment, the signal processing unit is configured to process the reflection signal based on the cut-off threshold value to cut off a portion of the reflection signal corresponding to detected wave, such as light, optionally projected onto, and reflected from the abdominal compression equipment to get a processed reflection signal corresponding to detected wave, such as light, optionally projected onto, and reflected from a surface of an abdominal and chest region of the patient.

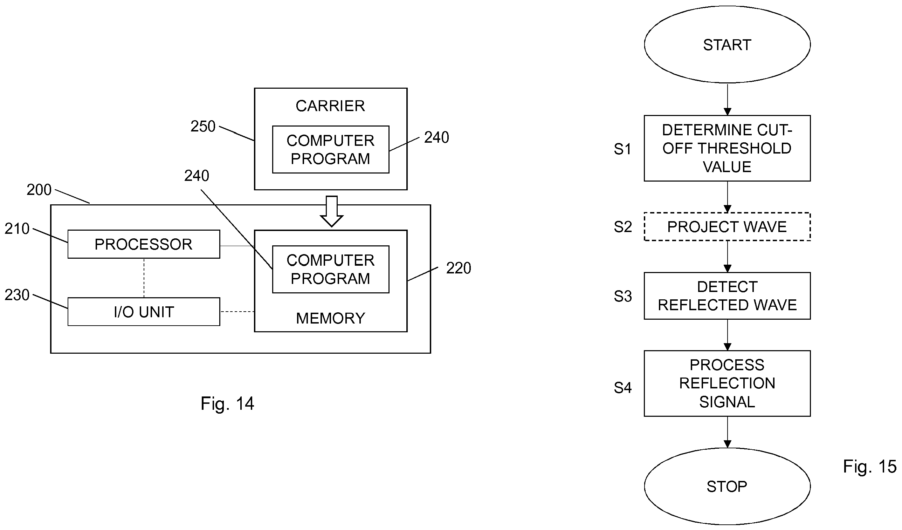

Fig. 15 is a flow chart illustrating a patient monitoring method according to an embodiment. The method comprises determining, in step S1, a cut-off threshold value based on a signal representing a reflection response, such as a color or tone, of a skin of a patient and a signal representing a reflection response, such as a color or tone, of a patient covering object covering at least a portion of a surface of the patient. The method optionally comprises projecting a wave, such as light, onto at least a portion of the patient in the optional step S2. Step S3 comprises detecting a wave, such as light, reflected from at least a portion of the patient and generating a reflection signal based on the detected wave, such as light. The method further comprises processing, in step S4, the reflection signal based on the cut-off threshold value to cut off a portion of the reflection signal corresponding to detected wave, such as light, reflected from the patient covering object to get a processed reflection signal corresponding to detected wave, such as light, reflected from the surface of the patient.

It will be appreciated that the methods, method steps and units, unit functions described herein can be implemented, combined and re-arranged in a variety of ways.

For example, embodiments may be implemented in hardware, or in software for execution by suitable processing circuitry, or a combination thereof.

The steps, functions, procedures, modules and/or blocks described herein may be implemented in hardware using any conventional technology, such as discrete circuit or integrated circuit technology, including both general-purpose electronic circuitry and application-specific circuitry.

Alternatively, or as a complement, at least some of the steps, functions, procedures, modules and/or blocks described herein may be implemented in software such as a computer program for execution by suitable processing circuitry such as one or more processors or processing units.

Examples of processing circuitry includes, but is not limited to, one or more microprocessors, one or more Digital Signal Processors (DSPs), one or more Central Processing Units (CPUs), video acceleration hardware, and/or any suitable programmable logic circuitry such as one or more Field Programmable Gate Arrays (FPGAs), or one or more Programmable Logic Controllers (PLCs).

It should also be understood that it may be possible to re-use the general processing capabilities of any conventional device or unit in which the proposed technology is implemented. It may also be possible to re-use existing software, e.g., by reprogramming of the existing software or by adding new software components.

Fig. 11 is a schematic block diagram illustrating an example of a patient monitoring system 90 based on a processor-memory implementation according to an embodiment. In this particular example, the patient monitoring system 90 comprises a processor 91, such as processing circuitry, and a memory 92. The memory 92 comprises instructions executable by the processor 91.

In an embodiment, the processor 91 is operative to determine the cut-off threshold value based on the signal representing the reflection response, such as the color or tone, of the skin of the patient and the signal representing the reflection response, such as the color or tone, of the patient covering object. The processor 91 is also operative to process the reflection signal based on the cut-off threshold value to cut off a portion of the reflection signal corresponding to detected wave, such as light, reflected from the patient covering object to get the processed reflection signal corresponding to detected wave, such as light, reflected from the surface of the patient.

Optionally, the patient monitoring system 90 may also include a communication circuit, represented by an input/output (I/O) unit 93 in Fig. 11. The I/O unit 93 may include functions for wired and/or wireless communication with other units and devices in connection with a radiation therapy machine. The I/O unit 93 may be interconnected to the processor 91 and/or memory 92. By way of example, the I/O unit 93 may include any of the following: a receiver, a transmitter, a transceiver, I/O circuitry, input port(s) and/or output port(s).

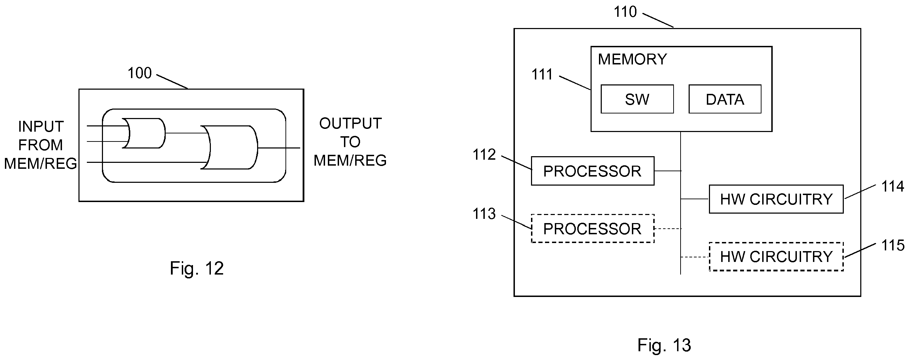

Fig. 12 is a schematic block diagram illustrating another example of a patient monitoring system 100 based on a hardware circuitry implementation according to an embodiment. Particular examples of suitable hardware circuitry include one or more suitably configured or possibly reconfigurable electronic circuitry, e.g., Application Specific Integrated Circuits (ASICs), FPGAs, or any other hardware logic such as circuits based on discrete logic gates and/or flip-flops interconnected to perform specialized functions in connection with suitable registers (REG), and/or memory units (MEM).

Fig. 13 is a schematic block diagram illustrating yet another example of a patient monitoring system 110 based on combination of both processor(s) 112, 113 and hardware circuitry 114, 115 in connection with suitable memory unit(s) 111. The patient monitoring system 110 comprises one or more processors 112, 113, memory 111 including storage for software (SW) and data, and one or more units of hardware circuitry 114, 115. The overall functionality is thus partitioned between programmed software for execution on one or more processors 112, 113, and one or more pre-configured or possibly reconfigurable hardware circuits 114, 115. The actual hardware-software partitioning can be decided by a system designer based on a number of factors including processing speed, cost of implementation and other requirements.

Fig. 14 is a schematic diagram illustrating an example of a computer program based implementation of a patient monitoring system 200 according to an embodiment. In this particular example, at least some of the steps, functions, procedures, modules and/or blocks described herein are implemented in a computer program 240, which is loaded into the memory 220 for execution by processing circuitry including one or more processors 210. The processor(s) 210 and memory 220 are interconnected to each other to enable normal software execution. An optional I/O unit 230 may also be interconnected to the processor(s) 210 and/or the memory 220 to enable input and/or output of relevant data.

The term 'processor' should be interpreted in a general sense as any circuitry, system or device capable of executing program code or computer program instructions to perform a particular processing, determining or computing task.

The processing circuitry including one or more processors 210 is thus configured to perform, when executing the computer program 240, well-defined processing tasks such as those described herein.

The processing circuitry does not have to be dedicated to only execute the above-described steps, functions, procedure and/or blocks, but may also execute other tasks.

In a particular embodiment, the computer program 240 comprises instructions, which when executed by at least one processor 210, cause the at least one processor 210 to determine a cut-off threshold value based on a signal representing a reflection response, such as a color or tone, of a skin of a patient and a signal representing a reflection response, such as a color or tone, of a patient covering object covering at least a portion of a surface of said patient. The at least one processor 210 is also caused to process a reflection signal, representing a detected wave, such as light, reflected from at least a portion of the patient, based on the cut-off threshold value to cut off a portion of the reflection signal corresponding to detected wave, such as light, reflected from the patient covering object to get a processed reflection signal corresponding to detected wave, such as light, reflected from the surface of the patient.

The proposed technology also provides a carrier 250 comprising the computer program 240. The carrier 250 is one of an electronic signal, an optical signal, an electromagnetic signal, a magnetic signal, an electric signal, a radio signal, a microwave signal, or a computer-readable storage medium.

By way of example, the software or computer program 240 may be realized as a computer program product, which is normally carried or stored on a computer-readable medium 250, in particular a non-volatile medium. The computer-readable medium may include one or more removable or non-removable memory devices including, but not limited to a Read-Only Memory (ROM), a Random Access Memory (RAM), a Compact Disc (CD), a Digital Versatile Disc (DVD), a Blu-ray disc, a Universal Serial Bus (USB) memory, a Hard Disk Drive (HDD) storage device, a flash memory, a magnetic tape, or any other conventional memory device. The computer program 240 may thus be loaded into the operating memory 220 of a patient monitoring system 200 for execution by the processing circuitry 210 thereof.

The flow diagram or diagrams presented herein may be regarded as a computer flow diagram or diagrams, when performed by one or more processors. A corresponding patient monitoring system may be defined as a group of function modules, where each step performed by the processor corresponds to a function module. In this case, the function modules are implemented as a computer program running on the processor.

The computer program residing in memory may, thus, be organized as appropriate function modules configured to perform, when executed by the processor, at least part of the steps and/or tasks described herein.

In such an embodiment, see Fig. 2, the patient monitoring system 70 comprises a threshold determining unit 71 for determining a cut-off threshold value based on a signal representing a reflection response, such as a color or tone, of a skin of a patient and a signal representing a reflection response, such as a color or tone, of a patient covering object covering at least a portion of a surface of said patient. The patient monitoring system 70 also comprises a signal processing unit 72 for processing a reflection signal, representing a detected wave, such as light, reflected from at least a portion of the patient, based on the cut-off threshold value to cut off a portion of the reflection signal corresponding to detected wave, such as light, reflected from the patient covering object to get a processed reflection signal corresponding to detected wave, such as light, reflected from the surface of the patient.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

**Claims**

1. A patient monitoring system (70) comprising:
   a signal processing unit (72) configured to process a reflection signal, representing a detected wave reflected from at least a portion of a patient (30) to cut off a portion of said reflection signal corresponding to detected wave reflected from a patient covering object (32) covering at least a portion of a surface of said patient (30) to get a processed reflection signal corresponding to detected wave reflected from said surface of said patient (30), **characterized in that** it further comprises: a threshold determining unit (71) configured to determine a cut-off threshold value based on a signal representing a reflection response of a skin of said patient (30) and a signal representing a reflection response of said patient covering object (32), wherein said signal processing unit (72) is configured to process said reflection signal based on said cut off threshold value to cut off said portion of said reflection signal corresponding to said detected wave reflected from said patient covering object to get said processed reflection signal.

2. The patient monitoring system according to claim 1, wherein
   said threshold determining unit (71) is configured to determine said cut-off threshold value based on a signal representing a color or tone of said skin of said patient (30) and a signal representing a color or tone of said patient covering object (32); and
   said signal processing unit (72) configured to process said reflection signal, representing detected light reflected from said at least a portion of said patient (30), based on said cut-off threshold value to cut off a portion of said reflection signal corresponding to detected light reflected from said patient covering object (32) to get said processed light signal corresponding to detected light reflected from said surface of said patient (30).

3. The patient monitoring system according to claim 1 or 2, further comprising:

   a detector (64) configured to detect said wave reflected from said at least a portion of said patient (30) and generate said reflection signal based on said detected wave; and
   an object unit (73) configured to generate said signal representing said reflection response of said patient covering object (32) based on a reflection signal generated by said detector (64) and representing detected wave reflected from said at least a portion of said patient covering object (32).

4. The patient monitoring system according to claim 3, wherein said detector (64) is configured to detect wave reflected from at least a portion of said patient covering object (32) and to generate said reflection signal based on said detected wave.

5. The patient monitoring system according to claim 3 or 4, further comprising:

   a screen (80) configured to display an image generated based on said reflection signal; and
   a selecting unit (75) configured to generate a selection signal representing a selected portion of said image based on activation of a user input (80, 82), wherein said object unit (73) is configured to generate said signal representing said reflection response of said patient covering object (32) based on a reflection signal generated by said detector (64) based on a reflection response of said portion of said image selected based on said selection signal.

6. The patient monitoring system according to any of the claims 3 to 5, wherein said object unit (73) is configured to identify, in an image generated based on said reflection signal, a portion of said image corresponding to said patient covering object (32) based on a reference image of said patient (30) and/or said patient covering object (32) and to generate said signal representing said reflection response of said patient covering object (32) based on a reflection response of said portion of said image identified based on said reference image.

7. The patient monitoring system according to any of the claims 1 to 6, further comprising:

   a detector (64) configured to detect said wave reflected from said at least a portion of said patient (30) and generate said reflection signal based on said detected wave; and
   a skin unit (74) configured to generate said signal representing said reflection response of said skin of said patient (30) based on a reflection signal generated by said detector (64) and representing detected wave reflected from said at least a portion of said patient (30).

8. The patient monitoring system according to claim 7, wherein said detector (64) is configured to detect wave reflected from said at least a portion of said patient (30) prior to covering said at least a portion of said patient (30) by said patient covering object (32) and to generate said reflection signal based on said detected wave.

9. The patient monitoring system according to claim 7 or 8, further comprising:

   a screen (80) configured to display an image generated based on said reflection signal; and
   a selecting unit (75) configured to generate a selection signal representing a selected portion of said image based on activation of a user input (80, 82), wherein said skin unit (74) is configured to generate said signal representing said reflection response of said skin of said patient (30) based on a reflection response of said selected portion of said image based on said selection signal.

10. The patient monitoring system according to any of the claims 7 to 9, wherein said skin unit (74) is configured to identify, in an image generated based on said reflection signal, a portion of said image corresponding to said skin of said patient (30) based on a reference image of said patient (30) and/or of said patient covering object (32) and to generate said signal representing said reflection response of said skin of said patient (30) based on a reflection response of said portion of said image identified based on said reference image.

11. The patient monitoring system according to claim 1 or 2, further comprising an object unit (73) configured to generate said signal representing said reflection response of said patient covering object (32) as representing a reflection response selected from a set of multiple predefined reflection responses based on activation of a user input (80, 82).

12. The patient monitoring system according to any of the claims 1 to 6, further comprising a skin unit (74) configured to generate said signal representing said reflection response of said skin of said patient (30) as representing a reflection response selected from a set of multiple predefined reflection responses based on activation of said user input (80, 82).

13. The patient monitoring system according to any of the claims 1 to 12, wherein
   said signal processing unit (72) is configured to process said reflection signal based on said cut-off threshold value and a signal representing a region of interest to cut off a portion of said reflection signal corresponding to detected wave reflected from said patient covering object (32) to get said processed reflection signal corresponding to detected wave reflected from said surface of said patient (30); and
   said signal representing said region of interest defines a region of said patient (30) comprising said surface of said patient (30).

14. The patient monitoring system according to any of the claims 1 to 13, further comprising:

   a patient surface generating unit (76) configured to generate a signal representing at least a portion of said surface of said patient (30) based on said processed reflection signal;
   a patient monitoring unit (78) configured to generate a difference signal based on a comparison between said signal representing said at least a portion of said surface of said patient (30) and a reference signal representing a reference surface; and
   a source controller (77) connected to said patient monitoring unit (78) and a radiation source (10) configured to irradiate a target volume (35) within said patient (30), said source controller (77) is configured to control irradiation from said radiation source (10) based on said difference signal.

15. A patient monitoring method comprising the steps of:

   detecting (S3) a wave reflected from at least a portion of a patient (30) and generating a reflection signal based on said detected wave; and processing (S4) said reflection signal to cut off a portion of said reflection signal corresponding to detected wave reflected from a patient covering object (32) covering at least a portion of a surface of said patient (30) to get a processed reflection signal corresponding to detected wave reflected from onto said surface of said patient (30), **characterized in that** it further comprises the step of
   determining (S1) a cut-off threshold value based on a signal representing a reflection response of a skin of said patient (30) and a signal representing a reflection response of said patient covering object (32), wherein processing (S4) said reflection signal comprises processing (S4) said reflection signal based on said cut-off threshold value to cut off said portion of said reflection signal corresponding to said detected wave reflected from said patient covering object (32) to get said processed reflection signal.

16. A computer program comprising instructions, which when executed by at least one processor (210), cause said at least one processor (210) to process a reflection signal, representing a detected wave reflected from at least a portion of a patient (30) to cut off a portion of said reflection signal corresponding to detected wave reflected from a patient covering object (32) covering at least a portion of a surface of said patient (30) to get a processed reflection signal corresponding to detected wave reflected from said surface of said patient (30), **characterized in that** said instructions cause said at least one processor (210) to determine a cut-off threshold value based on a signal representing a reflection response of a skin of a patient (30) and a signal representing a reflection response of said patient covering object (32) and process said reflection signal based on said cut-off threshold value to cut off said portion of said reflection signal corresponding to detected wave reflected from said patient covering object (32) to get said processed reflection signal.

17. A computer-readable storage medium (250) comprising a computer program (240) according to claim 16.

**Patentansprüche**

1. Patientenüberwachungssystem (70), umfassend:
   eine Signalverarbeitungseinheit (72), die zur Verarbeitung eines Reflexionssignals ausgelegt ist, das eine nachgewiesene Welle repräsentiert, die von zumindest einem Teil eines Patienten (30) reflektiert wurde, um einen Teil des Reflexionssignals abzuschalten, der der nachgewiesenen Welle entspricht, die von einem einen Patienten abdeckenden Gegenstand (32), der zumindest einen Teil einer Oberfläche des Patienten (30) abdeckt, reflektiert wurde, um ein verarbeitetes Reflexionssignal zu erhalten, das der nachgewiesenen Welle entspricht, die von der Oberfläche des Patienten (30) reflektiert wurde, **dadurch gekennzeichnet, dass** es ferner das Folgende umfasst:
   eine Schwellenwertbestimmungseinheit (71), die zur Bestimmung eines Abschaltschwellenwerts auf Basis eines Signals, das eine Reflexionsreaktion einer Haut des Patienten (30) repräsentiert, und eines Signals, das eine Reflexionsreaktion des den Patienten abdeckenden Gegenstands (32) repräsentiert, ausgelegt ist, wobei die Signalverarbeitungseinheit (72) zum Verarbeiten des Reflexionssignals auf Basis des Abschaltschwellenwerts ausgelegt ist, um den Teil des Reflexionssignals abzuschalten, der der nachgewiesenen Welle entspricht, die von dem den Patienten abdeckenden Gegenstand reflektiert wurde, um das verarbeitete Reflexionssignal zu erhalten.

2. Patientenüberwachungssystem nach Anspruch 1, wobei
   die Schwellenwertbestimmungseinheit (71) zur Bestimmung des Abschaltschwellenwerts auf Basis eines Signals, das eine Farbe oder einen Ton der Haut des Patienten (30) repräsentiert, und eines Signals, das eine Farbe oder einen Ton des den Patienten abdeckenden Gegenstands (32) repräsentiert, ausgelegt ist; und
   die Signalverarbeitungseinheit (72) zum Verarbeiten des Reflexionssignals, das nachgewiesenes Licht repräsentiert, das von dem zumindest einen Teil des Patienten (30) reflektiert wurde, auf Basis des Abschaltschwellenwerts ausgelegt ist, um einen Teil des Reflexionssignals, der nachgewiesenem Licht entspricht, das von dem den Patienten abdeckenden Gegenstand (32) reflektiert wurde, abzuschalten, um das verarbeitete Lichtsignal zu erhalten, das nachgewiesenem Licht entspricht, das von der Oberfläche des Patienten (30) reflektiert wurde.

3. Patientenüberwachungssystem nach Anspruch 1 oder 2, ferner umfassend:

   einen Detektor (64), der zum Nachweisen der Welle, die von dem zumindest einen Teil des Patienten (30) reflektiert wurde, und zum Erzeugen des Reflexionssignals auf Basis der nachgewiesenen Welle ausgelegt ist; und
eine Objekteinheit (73), die zum Erzeugen des Signals, das die Reflexionsreaktion des den Patienten abdeckenden Gegenstands (32) repräsentiert, auf Basis eines Reflexionssignals, das vom Detektor (64) erzeugt wurde und die nachgewiesene Welle repräsentiert, die von dem zumindest einen Teil des den Patienten abdeckenden Gegenstands (32) reflektiert wurde, ausgelegt ist.

4. Patientenüberwachungssystem nach Anspruch 3, wobei der Detektor (64) zum Nachweisen einer Welle, die von zumindest einem Teil des den Patienten abdeckenden Gegenstands (32) reflektiert wurde, und zum Erzeugen des Reflexionssignals auf Basis der nachgewiesenen Welle ausgelegt ist.

5. Patientenüberwachungssystem nach Anspruch 3 oder 4, ferner umfassend:

einen Bildschirm (80), der zum Anzeigen eines Bilds ausgelegt ist, das auf Basis des Reflexionssignals erzeugt wurde; und
eine Auswahleinheit (75), die zum Erzeugen eines Auswahlsignals, das einen ausgewählten Teil des Bilds repräsentiert, auf Basis einer Aktivierung einer Benutzereingabe (80, 82) ausgelegt ist, wobei die Objekteinheit (73) zum Erzeugen des Signals, das die Reflexionsreaktion des den Patienten abdeckenden Gegenstands (32) repräsentiert, auf Basis eines Reflexionssignals ausgelegt ist, das vom Detektor (64) auf Basis einer Reflexionsreaktion des Teils des Bilds, der auf Basis des Auswahlsignals ausgewählt wurde, erzeugt wurde.

6. Patientenüberwachungssystem nach einem der Ansprüche 3 bis 5, wobei die Objekteinheit (73) zum Identifizieren, in einem auf Basis des Reflexionssignals erzeugten Bild, eines Teils des Bilds, der dem den Patienten abdeckenden Gegenstand (32) entspricht, auf Basis eines Bezugsbilds des Patienten (30) und/oder des den Patienten abdeckenden Gegenstands (32), und zum Erzeugen des Signals, das die Reflexionsreaktion des den Patienten abdeckenden Gegenstands (32) repräsentiert, auf Basis einer Reflexionsreaktion des Teils des Bilds, der basierend auf dem Bezugsbild identifiziert wurde, ausgelegt ist.

7. Patientenüberwachungssystem nach einem der Ansprüche 1 bis 6, ferner umfassend:

einen Detektor (64), der zum Nachweisen der Welle, die von dem zumindest einen Teil des Patienten (30) reflektiert wurde, und zum Erzeugen des Reflexionssignals auf Basis der nachgewiesenen Welle ausgelegt ist; und
eine Hauteinheit (74), die zum Erzeugen des Signals, das die Reflexionsreaktion der Haut des Patienten (30) repräsentiert, auf Basis eines Reflexionssignals, das vom Detektor (64) erzeugt wurde und die nachgewiesene Welle repräsentiert, die von dem zumindest einen Teil des Patienten (30) reflektiert wurde, ausgelegt ist.

8. Patientenüberwachungssystem nach Anspruch 7, wobei der Detektor (64) zum Nachweisen einer Welle, die von dem zumindest einen Teil des Patienten (30) reflektiert wurde, vor dem Abdecken des zumindest einen Teils des Patienten (30) mit dem den Patienten abdeckenden Gegenstand (32), und zum Erzeugen des Reflexionssignals auf Basis der nachgewiesenen Welle ausgelegt ist.

9. Patientenüberwachungssystem nach Anspruch 7 oder 8, ferner umfassend:

einen Bildschirm (80), der zum Anzeigen eines Bilds ausgelegt ist, das auf Basis des Reflexionssignals erzeugt wurde; und
eine Auswahleinheit (75), die zum Erzeugen eines Auswahlsignals, das einen ausgewählten Teil des Bilds repräsentiert, auf Basis einer Aktivierung einer Benutzereingabe (80, 82) ausgelegt ist, wobei die Hauteinheit (74) zum Erzeugen des Signals, das die Reflexionsreaktion der Haut des Patienten (30) repräsentiert, auf Basis einer Reflexionsreaktion des ausgewählten Teils des Bilds auf Basis des Auswahlsignals ausgelegt ist.

10. Patientenüberwachungssystem nach einem der Ansprüche 7 bis 9, wobei die Hauteinheit (74) zum Identifizieren, in einem auf Basis des Reflexionssignals erzeugten Bild, eines Teils des Bilds, der der Haut des Patienten (30) entspricht, auf Basis eines Bezugsbilds des Patienten (30) und/oder des den Patienten abdeckenden Gegenstands (32), und zum Erzeugen des Signals, das die Reflexionsreaktion der Haut des Patienten (30) repräsentiert, auf Basis einer Reflexionsreaktion des Teils des Bilds, der basierend auf dem Bezugsbild identifiziert wurde, ausgelegt ist.

11. Patientenüberwachungssystem nach Anspruch 1 oder 2, ferner umfassend eine Objekteinheit (73), die zum Erzeugen des Signals, das die Reflexreaktion des den Patienten abdeckenden Gegenstands (32) repräsentiert, als Repräsentation einer Reflexionsreaktion, die aus einem Satz von mehreren vordefinierten Reflexionsreaktionen auf Basis einer Aktivierung einer Benutzereingabe (80, 82) ausgewählt wurde, ausgelegt ist.

12. Patientenüberwachungssystem nach einem der Ansprüche 1 bis 6, ferner umfassend eine Hauteinheit (74), die zum Erzeugen des Signals, das die Reflexreaktion der Haut des Patienten (30) repräsentiert, als Repräsentation einer Reflexionsreaktion, die aus einem Satz von mehreren vordefinierten Reflexionsreaktionen auf Basis einer Aktivierung der Benutzereingabe (80, 82) ausgewählt wurde, ausgelegt ist.

13. Patientenüberwachungssystem nach einem der Ansprüche 1 bis 12, wobei
die Signalverarbeitungseinheit (72) zum Verarbeiten des Reflexionssignals auf Basis des Abschaltschwellenwerts und eines Signals, das eine interessierende Region repräsentiert, ausgelegt ist, zum Abschalten eines Teils des Reflexionssignals, der der nachgewiesenen Welle entspricht, die von dem den Patienten abdeckenden Gegenstand (32) reflektiert wurde, um das verarbeitete Reflexionssignal zu erhalten, das der nachgewiesenen Welle entspricht, die von der Oberfläche des Patienten (30) reflektiert wurde; und
das die interessierende Region repräsentierende Signal eine Region des Patienten (30) definiert, die die Oberfläche des Patienten (30) umfasst.

14. Patientenüberwachungssystem nach einem der Ansprüche 1 bis 13, ferner umfassend:

eine Patientenoberflächenerzeugungseinheit (76), die zum Erzeugen eines Signals, das zumindest einen Teil der Oberfläche des Patienten (30) repräsentiert, auf Basis des verarbeiteten Reflexionssignals ausgelegt ist;
eine Patientenüberwachungseinheit (78), die zum Erzeugen eines Differenzsignals auf Basis eines Vergleichs zwischen dem Signal, das den zumindest einen Teil der Oberfläche des Patienten (30) repräsentiert, und einem eine Bezugsoberfläche repräsentierenden Referenzsignal ausgelegt ist; und
eine Quellensteuerung (77), die mit der Patientenüberwachungseinheit (78) und einer Strahlungsquelle (10) verbunden ist, die zum Bestrahlen eines Zielvolumens (35) im Patienten (30) ausgelegt ist, wobei die Quellensteuerung (77) zur Steuerung der Bestrahlung von der Bestrahlungsquelle (10) auf Basis des Differenzsignals ausgelegt ist.

15. Patientenüberwachungsverfahren, die folgenden Schritte umfassend:

Nachweisen (S3) einer Welle, die von zumindest einem Teil eines Patienten (30) reflektiert wurde, und Erzeugen eines Reflexionssignals auf Basis der nachgewiesenen Welle; und
Verarbeiten (S4) des Reflexionssignals, um einen Teil des Reflexionssignals abzuschalten, der der nachgewiesenen Welle entspricht, die von einem einen Patienten abdeckenden Gegenstand (32), der zumindest einen Teil einer Oberfläche des Patienten (30) abdeckt, reflektiert wurde, um ein verarbeitetes Reflexionssignal zu erhalten, das der nachgewiesenen Welle entspricht, die von der Oberfläche des Patienten (30) reflektiert wurde, **dadurch gekennzeichnet, dass** es ferner den Schritt der Bestimmung (S1) eines Abschaltschwellenwerts auf Basis eines Signals, das eine Reflexionsreaktion einer Haut des Patienten (30) repräsentiert, und eines Signals, das eine Reflexionsreaktion des den Patienten abdeckenden Gegenstands (32) repräsentiert, umfasst, wobei das Verarbeiten (S4) des Reflexionssignals das Verarbeiten (S4) des Reflexionssignals auf Basis des Abschaltschwellenwerts umfasst, um den Teil des Reflexionssignals abzuschalten, der der nachgewiesenen Welle entspricht, die von dem den Patienten abdeckenden Gegenstand (32) reflektiert wurde, um das verarbeitete Reflexionssignal zu erhalten.

16. Computerprogramm, das Anweisungen umfasst, die bei Ausführung durch mindestens einen Prozessor (210) veranlassen, dass der mindestens eine Prozessor (210)
ein Reflexionssignal verarbeitet, das eine nachgewiesene Welle repräsentiert, die von zumindest einem Teil eines Patienten (30) reflektiert wurde, um einen Teil des Reflexionssignals abzuschalten, der der nachgewiesenen Welle entspricht, die von einem einen Patienten abdeckenden Gegenstand (32), der zumindest einen Teil einer Oberfläche des Patienten (30) abdeckt, reflektiert wurde, um ein verarbeitetes Reflexionssignal zu erhalten, das der nachgewiesenen Welle entspricht, die von der Oberfläche des Patienten (30) reflektiert wurde, **dadurch gekennzeichnet, dass** die Anweisungen den mindestens einen Prozessor (210) veranlassen, einen Abschaltschwellenwert auf Basis eines Signals, das eine Reflexionsreaktion einer Haut eines Patienten (30) repräsentiert, und eines Signals, das eine Reflexionsreaktion des den Patienten abdeckenden Gegenstands (32) repräsentiert, zu bestimmen und das Reflexionssignal auf Basis des Abschaltschwellenwerts zu verarbeiten, um den Teil des Reflexionssignals abzuschalten, der der nachgewiesenen Welle entspricht, die von dem den Patienten abdeckenden Gegenstand (32) reflektiert wurde, um das verarbeitete Reflexionssignal zu erhalten.

17. Computerlesbares Speichermedium (250), das ein Computerprogramm (240) nach Anspruch 16 umfasst.

**Revendications**

1. Système de surveillance de patient (70) comprenant :
une unité de traitement de signal (72) configurée pour traiter un signal de réflexion, représentant une onde détectée réfléchie par au moins une partie d'un patient (30) pour couper une partie dudit signal de réflexion correspondant à l'onde détectée réfléchie par un objet de couverture de patient (32) couvrant au moins une partie d'une surface dudit patient (30) pour obtenir un signal de réflexion traité correspondant à l'onde détectée réfléchie par ladite surface dudit patient (30), **caractérisé en ce qu'**il comprend en outre :
une unité de détermination de seuil (71) configurée pour déterminer une valeur de seuil de coupure sur la base d'un signal représentant une réponse de réflexion d'une peau dudit patient (30) et d'un signal représentant une réponse de réflexion dudit objet de couverture de patient (32), ladite unité de traitement de signal (72) étant configurée pour traiter ledit signal de réflexion sur la base de ladite valeur de seuil de coupure pour couper ladite partie dudit signal de réflexion correspondant à ladite onde détectée réfléchie par ledit objet de couverture de patient pour obtenir ledit signal de réflexion traité.

2. Système de surveillance de patient selon la revendication 1,
ladite unité de détermination de seuil (71) étant configurée pour déterminer ladite valeur de seuil de coupure sur la base d'un signal représentant une couleur ou un ton de ladite peau dudit patient (30) et d'un signal représentant une couleur ou un ton dudit objet de couverture de patient (32) ; et
ladite unité de traitement de signal (72) étant configurée pour traiter ledit signal de réflexion, représentant la lumière détectée réfléchie par ladite au moins une partie dudit patient (30), sur la base de ladite valeur de seuil de coupure pour couper une partie dudit signal de réflexion correspondant à la lumière détectée réfléchie par ledit objet de couverture de patient (32) pour obtenir ledit signal de lumière traité correspondant à la lumière détectée réfléchie par ladite surface dudit patient (30).

3. Système de surveillance de patient selon la revendication 1 ou 2, comprenant en outre :

un détecteur (64) configuré pour détecter ladite onde réfléchie par ladite au moins une partie dudit patient (30) et générer ledit signal de réflexion sur la base de ladite onde détectée ; et
une unité d'objet (73) configurée pour générer ledit signal représentant ladite réponse de réflexion dudit objet de couverture de patient (32) sur la base d'un signal de réflexion généré par ledit détecteur (64) et représentant l'onde détectée réfléchie par ladite au moins une partie dudit objet de couverture de patient (32).

4. Système de surveillance de patient selon la revendication 3, ledit détecteur (64) étant configuré pour détecter une onde réfléchie par au moins une partie dudit objet de couverture de patient (32) et pour générer ledit signal de réflexion sur la base de ladite onde détectée.

5. Système de surveillance de patient selon la revendication 3 ou 4, comprenant en outre :

un écran (80) configuré pour afficher une image générée sur la base dudit signal de réflexion ; et
une unité de sélection (75) configurée pour générer un signal de sélection représentant une partie sélectionnée de ladite image sur la base de l'activation d'une entrée utilisateur (80, 82), ladite unité d'objet (73) étant configurée pour générer ledit signal représentant ladite réponse de réflexion dudit objet de couverture de patient (32) sur la base d'un signal de réflexion généré par ledit détecteur (64) sur la base d'une réponse de réflexion de ladite partie de ladite image sélectionnée sur la base dudit signal de sélection.

6. Système de surveillance de patient selon l'une quelconque des revendications 3 à 5, ladite unité d'objet (73) étant configurée pour identifier, dans une image générée sur la base dudit signal de réflexion, une partie de ladite image correspondant audit objet de couverture de patient (32) sur la base d'une image de référence dudit patient (30) et/ou dudit objet de couverture de patient (32) et pour générer ledit signal représentant ladite réponse de réflexion dudit objet de couverture de patient (32) sur la base d'une réponse de réflexion de ladite partie de ladite image identifiée sur la base de ladite image de référence.

7. Système de surveillance de patient selon l'une quelconque des revendications 1 à 6, comprenant en outre :

un détecteur (64) configuré pour détecter ladite onde réfléchie par ladite au moins une partie dudit patient (30) et générer ledit signal de réflexion sur la base de ladite onde détectée ; et
une unité de peau (74) configurée pour générer ledit signal représentant ladite réponse de réflexion de ladite peau dudit patient (30) sur la base d'un signal de réflexion généré par ledit détecteur (64) et représentant l'onde détectée réfléchie par ladite au moins une partie dudit patient (30).

8. Système de surveillance de patient selon la revendication 7, ledit détecteur (64) étant configuré pour détecter une onde réfléchie par ladite au moins une partie dudit patient (30) avant de couvrir ladite au moins une partie dudit patient (30) par ledit objet de couverture de patient (32) et pour générer ledit signal de réflexion sur la base de ladite onde détectée.

9. Système de surveillance de patient selon la revendication 7 ou 8, comprenant en outre :

   un écran (80) configuré pour afficher une image générée sur la base dudit signal de réflexion ; et une unité de sélection (75) configurée pour générer un signal de sélection représentant une partie sélectionnée de ladite image sur la base de l'activation d'une entrée utilisateur (80, 82), ladite unité de peau (74) étant configurée pour générer ledit signal représentant ladite réponse de réflexion de ladite peau dudit patient (30) sur la base d'une réponse de réflexion de ladite partie sélectionnée de ladite image sur la base dudit signal de sélection.

10. Système de surveillance de patient selon l'une quelconque des revendications 7 à 9, ladite unité de peau (74) étant configurée pour identifier, dans une image générée sur la base dudit signal de réflexion, une partie de ladite image correspondant à ladite peau dudit patient (30) sur la base d'une image de référence dudit patient (30) et/ou dudit objet de couverture de patient (32) et pour générer ledit signal représentant ladite réponse de réflexion de ladite peau dudit patient (30) sur la base d'une réponse de réflexion de ladite partie de ladite image identifiée sur la base de ladite image de référence.

11. Système de surveillance de patient selon la revendication 1 ou 2, comprenant en outre une unité d'objet (73) configurée pour générer ledit signal représentant ladite réponse de réflexion dudit objet de couverture de patient (32) comme représentant une réponse de réflexion sélectionnée à partir d'un ensemble de multiples réponses de réflexion prédéfinies sur la base de l'activation d'une entrée d'utilisateur (80, 82).

12. Système de surveillance de patient selon l'une quelconque des revendications 1 à 6, comprenant en outre une unité de peau (74) configurée pour générer ledit signal représentant ladite réponse de réflexion de ladite peau dudit patient (30) comme représentant une réponse de réflexion sélectionnée à partir d'un ensemble de multiples réponses de réflexion prédéfinies sur la base de l'activation de ladite entrée utilisateur (80, 82).

13. Système de surveillance de patient selon l'une quelconque des revendications 1 à 12, ladite unité de traitement de signal (72) étant configurée pour traiter ledit signal de réflexion sur la base de ladite valeur de seuil de coupure et d'un signal représentant une région d'intérêt pour couper une partie dudit signal de réflexion correspondant à l'onde détectée réfléchie par ledit objet de couverture de patient (32) pour obtenir ledit signal de réflexion traité correspondant à l'onde détectée réfléchie par ladite surface dudit patient (30) ; et ledit signal représentant ladite région d'intérêt définissant une région dudit patient (30) comprenant ladite surface dudit patient (30).

14. Système de surveillance de patient selon l'une quelconque des revendications 1 à 13, comprenant en outre :

   une unité de génération de surface de patient (76) configurée pour générer un signal représentant au moins une partie de ladite surface dudit patient (30) sur la base dudit signal de réflexion traité ; une unité de surveillance de patient (78) configurée pour générer un signal de différence sur la base d'une comparaison entre ledit signal représentant ladite au moins une partie de ladite surface dudit patient (30) et un signal de référence représentant une surface de référence ; et un dispositif de commande de source (77) connecté à ladite unité de surveillance de patient (78) et à une source de rayonnement (10) configurée pour irradier un volume cible (35) à l'intérieur dudit patient (30), ledit dispositif de commande de source (77) étant configuré pour commander l'irradiation à partir de ladite source de rayonnement (10) sur la base dudit signal de différence.

15. Procédé de surveillance d'un patient comprenant les étapes de :

   détection (S3) d'une onde réfléchie par au moins une partie d'un patient (30) et génération d'un signal de réflexion sur la base de ladite onde détectée ; et traitement (S4) dudit signal de réflexion pour couper une partie dudit signal de réflexion correspondant à une onde détectée réfléchie par un objet de couverture de patient (32) couvrant au moins une partie d'une surface dudit patient (30) pour obtenir un signal de réflexion traité correspondant à l'onde détectée réfléchie par ladite surface dudit patient (30), **caractérisé en ce qu'**il comprend en outre l'étape de

détermination (S1) d'une valeur de seuil de coupure sur la base d'un signal représentant une réponse de réflexion d'une peau dudit patient (30) et d'un signal représentant une réponse de réflexion dudit objet de couverture de patient (32), le traitement (S4) dudit signal de réflexion comprenant le traitement (S4) dudit signal de réflexion sur la base de ladite valeur de seuil de coupure pour couper ladite partie dudit signal de réflexion correspondant à ladite onde détectée réfléchie par ledit objet de couverture de patient (32) pour obtenir ledit signal de réflexion traité.

16. Programme informatique comprenant des instructions qui, lorsqu'elles sont exécutées par au moins un processeur (210), amènent ledit au moins un processeur (210) à traiter un signal de réflexion, représentant une onde détectée réfléchie par au moins une partie d'un patient (30) pour couper une partie dudit signal de réflexion correspondant à l'onde détectée réfléchie par un objet de couverture de patient (32) couvrant au moins une partie d'une surface dudit patient (30) pour obtenir un signal de réflexion traité correspondant à l'onde détectée réfléchie par ladite surface dudit patient (30), **caractérisé en ce que** lesdites instructions amènent ledit au moins un processeur (210) à déterminer une valeur de seuil de coupure sur la base d'un signal représentant une réponse de réflexion d'une peau d'un patient (30) et d'un signal représentant une réponse de réflexion dudit objet de couverture de patient (32), et à traiter ledit signal de réflexion sur la base de ladite valeur de seuil de coupure pour couper ladite partie dudit signal de réflexion correspondant à l'onde détectée réfléchie par ledit objet de couverture de patient (32) pour obtenir ledit signal de réflexion traité.

17. Support de stockage lisible par ordinateur (250) comprenant un programme informatique (240) selon la revendication 16.

Fig. 1

EP 3 395 408 B1

CONTROL
SIGNALS

THRESHOLD
DETERMINING
UNIT

SIGNAL
PROCESSING
UNIT

70

71

72

80

64

62

60

Fig. 2

**Fig. 3**

**Fig. 4**

**Fig. 5**

| |
|---|
| **70** |
| THRESHOLD DETERMINING UNIT — 71 |
| SIGNAL PROCESSING UNIT — 72 |
| PATIENT SURFACE GENERATING UNIT — 76 |
| SOURCE CONTROLLER — 77 |

CONTROL SIGNALS

80

**Fig. 6**

| |
|---|
| **70** |
| THRESHOLD DETERMINING UNIT — 71 |
| SIGNAL PROCESSING UNIT — 72 |
| PATIENT SURFACE GENERATING UNIT — 76 |
| PATIENT MONITORING UNIT — 78 |
| SOURCE CONTROLLER — 77 |

CONTROL SIGNALS

80

EP 3 395 408 B1

Fig. 7

SIGNAL
AMPLITUDE

SKIN

HOLES IN
MASK

MASK

T

DISTANCE

Fig. 9

SIGNAL
AMPLITUDE

SKIN

HOLES IN
MASK

MASK

DISTANCE

Fig. 8

Fig. 10

Fig. 11

EP 3 395 408 B1

Fig. 13

Fig. 12

## Fig. 14

CARRIER

250 — COMPUTER
PROGRAM — 240

200

240

210 — PROCESSOR

230 — I/O UNIT

COMPUTER
PROGRAM — 220

MEMORY

Fig. 14

## Fig. 15

START

S1 — DETERMINE CUT-OFF THRESHOLD VALUE

S2 — PROJECT WAVE

S3 — DETECT REFLECTED WAVE

S4 — PROCESS REFLECTION SIGNAL

STOP

Fig. 15

EP 3 395 408 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7024237 B
- US 20110137102 A
- US 20160155006 A
- US 8235530 B
- US 6919838 B
- US 9468395 B
- US 20160081618 A
- US 20100185102 A
- US 20150320342 A

**Non-patent literature cited in the description**

- *Journal of Applied Clinical Medical Physics,* 2010, vol. 11 (3), 26-37